(19) Europäisches Patentamt
European Patent Office
Office européen des brevets

(11) **EP 2 896 956 A1**

(12) **EUROPEAN PATENT APPLICATION**

(43) Date of publication:
**22.07.2015 Bulletin 2015/30**

(51) Int Cl.:
*G01N 21/552* (2014.01)          *G01N 21/65* (2006.01)

(21) Application number: **15151213.4**

(22) Date of filing: **15.01.2015**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **15.01.2014 EP 14151295**

(71) Applicants:
• **IMEC VZW**
  **3001 Leuven (BE)**
• **Panasonic Corporation**
  **Osaka 571-8501 (JP)**
• **Katholieke Universiteit Leuven**
  **3000 Leuven (BE)**

(72) Inventors:
• **Okumura, YASUAKI**
  **3001 Leuven (BE)**
• **Jans, Hilde**
  **3001 Leuven (BE)**
• **Li, Jiaqi**
  **3001 Leuven (BE)**
• **Minamiguchi, Masaru**
  **3001 Leuven (BE)**
• **Lagae, Liesbet**
  **3001 Leuven (BE)**

(74) Representative: **Clerix, André**
  **IMEC vzw**
  **IP Department**
  **Kapeldreef 75**
  **3001 Leuven (BE)**

(54) **Implantable SERS sensing device and method to fabricate**

(57)    A method for manufacturing a SERS sensing device (100), the method comprising: providing a silicon substrate (101), creating a silane layer (102) on a surface of the silicon substrate (101), and creating a plurality of plasmonic nanostructures (103) on the silane layer (102), characterized in that: creating a plurality of plasmonic nanostructures (103) on the silane layer (102) comprises completely covering the silane layer (102) with a metallic plasmonic layer (104). Further a SERS sensing device is presented which is implantable.

**FIG 3A**

**100**

EP 2 896 956 A1

## Description

### Field of the Disclosure

The field of the disclosure is related to biosensors. In particular, it is related to plasmonic biosensors. More in particular, it is related to implantable plasmonic biosensors.

### Background to the Disclosure

Surface enhanced Raman scattering (SERS) substrates are used for the identification of analytes. State of the art SERS substrates contain plasmonic nanostructures, e.g. gold nanostructures, which are located on a substrate, e.g. a glass substrate. A problem with these SERS substrates is the adhesion of the plasmonic nanostructures to the substrate. When using SERS substrates for implantation purposes, e.g. in the human or animal body, the adhesion of plasmonic nanostructures to the substrate becomes very important. When implanting the SERS substrate in the body, the plasmonic nanostructures peel off from the substrate. Also, after implantation plasmonic nanostructure are removed from the substrate due to the healing process of tissue or when the substrate is manipulated.

In Lamy de la Chapelle, "New Gold Nanoparticles Adhesion Process Opening the Way of Improved and Highly Sensitive Plasmonics Technologies", Plasmonics (2013), 8:411-415, a method of manufacturing a SERS substrate is described. The adhesion between gold nanostructures and a glass substrate is improved by using an adhesion layer that permits a covalent bonding between gold and glass. However, the adhesion strength is not sufficient for implantation purposes. Also, the sensitivity of the SERS substrate is reduced due to the fact that analytes bind to the adhesion layer which is present on the complete surface of the SERS substrate and hence exposed in between the gold nanostructures.

This disclosure provides a solution for the disadvantages described above.

### Summary of the Disclosure

In a **first** aspect of the disclosure, a method for manufacturing a SERS sensing device is presented comprising: providing a silicon substrate, creating a silane layer on a surface of the silicon substrate, and creating a plurality of plasmonic nanostructures on the silane layer, characterized in that: creating a plurality of plasmonic nanostructures on the silane layer comprises completely covering the silane layer with a metallic plasmonic layer.

According to an embodiment of the disclosure, creating the silane layer comprises immersing the substrate in a silane solution for 2,5 to 17 hours.

According to an embodiment of the disclosure, creating a plurality of plasmonic nanostructures further comprises: depositing a resist coating on the metallic plasmonic layer, thereafter lithographically patterning the resist coating, thereafter etching the metallic plasmonic layer and the silane layer using the patterned resist coating as a mask, and thereafter remove the patterned resist coating.

According to an embodiment of the disclosure, the method further comprises depositing receptor molecules on the surface of the silicon substrate, the receptor molecules being configured to bind to plasmonic nanostructures and to bind an analyte.

According to an embodiment of the disclosure, the analyte is glucose and the receptor molecules are boronic acid derivatives.

According to an embodiment of the disclosure, the silane layer is a mercapto-silane layer.

According to an embodiment of the disclosure, the metallic plasmonic layer is a gold, silver or copper layer.

According to an embodiment of the disclosure, the SERS sensing device is manufactured using CMOS compatible process steps.

A SERS sensing device is obtainable using the method as described above.

In a second aspect of the disclosure, an implantable SERS sensing device is presented comprising: a silicon substrate; a plurality of plasmonic nanostructures located on a surface of the silicon substrate, each plasmonic nanostructure comprising a silane layer and a metallic plasmonic metal layer; characterized in that: the silane layer and the metallic plasmonic layer of different plasmonic nanostructures are separated from each other, the silane layer thereby being only present in between the silicon substrate and the metallic plasmonic layer.

According to an embodiment of the disclosure, the adhesion strength between the metallic plasmonic layer of each plasmonic nanostructure and the silicon substrate is at least 44N/mm$^2$. The adhesion strength is measured using a commercially available scratch tool described below.

According to an embodiment of the disclosure, wherein the silane layer is a mercapto-silane layer.

According to an embodiment of the disclosure, a plasmonic nanostructure comprises a receptor molecule configured to bind an analyte.

According to an embodiment of the disclosure, the analyte is glucose and the receptor molecule is a boronic acid derivative.

According to an embodiment of the disclosure, the metallic plasmonic layer is a gold, silver or copper layer.

### Brief Description of the Drawings

In order to illustrate the disclosure schematic cross-sections of devices according to embodiments of the disclosure are provided. The cross-sections are not to scale. Throughout these schematic cross-sections the same hatching is used to identify similar features.

**FIG1A-1F** illustrates different steps of a method to fabricate a SERS substrate according to an embodiment of the disclosure

**FIG 2** illustrates the relation between gold adhesion strength and reaction time

**FIG 3A** illustrates a SERS substrate according to an embodiment of the disclosure

**FIG 3B** illustrates a SERS substrate with plasmonic nanostructures, each plasmonic nanostructure comprising receptor molecules for binding an analyte, according to an embodiment of the disclosure

**FIG 4A, B, C** illustrate bright field images of a SERS sensing device according to an embodiment of the disclosure after implantation in an animal for 2 months, magnified with different magnification factors

**FIG 5A, B, C** illustrate SEM images of a SERS sensing device according to an embodiment of the disclosure after implantation in an animal for 2 months, magnified with different magnification factors

**FIG 6** illustrates the attachment of molecules to plasmonic nanostructures

**FIG 7A, B** illustrate the measurement principle of the scratch tool to measure adhesion strength

**Description of the Disclosure**

It is an object of the disclosure to provide a reliable, robust and highly sensitive SERS substrate which can be implanted in the human or animal body.

It is an object of the disclosure to provide a SERS substrate which can be implanted and remain in the body for at least 2 months.

It is an object of the disclosure to provide a SERS substrate which can be implanted in the human or animal body and which does not provide any health hazards to the user.

It is an object of the disclosure to provide a technique to manufacture a SERS substrate which possesses all of the advantages described above.

It is an object of the disclosure, to provide a CMOS compatible method to manufacture a SERS substrate.

Throughout the description reference is made to a "silicon substrate". In embodiments, the term "silicon substrate" may include any substrate comprising silicon. For example, a silicon (Si) substrate, a silica (SiO2) substrate, a silicon nitride (SiN) substrate, a silicon germanium (SiGe) substrate or a glass silicon substrate. The term "silicon substrate" also includes silicon-on-glass, silicon-on-sapphire silicon substrates.

Throughout the description reference is made to "a receptor molecule" or "receptor molecules". This refers to one or more molecules which bind to a surface and are configured to bind a specific analyte. For example a receptor molecule may be a boronic acid derivative which binds to a surface of a material, e.g. a gold surface, and which is configured to bind a specific molecule, e.g. glucose molecules.

Throughout the description reference is made to "a metallic plasmonic layer". This refers to a metal layer which features plasmonic properties when radiated. The metal may be any metal suitable for performing plasmonic experiments.

The SERS sensing device presented in this disclosure may be used for in-vivo implantation. After implantation, plasmonic nanostructures present on the SERS substrate are vulnerable to external forces. These forces may be caused by the healing process of tissue which sets in immediately after implantation or by friction between the SERS substrate and biological tissue. As a result, plasmonic structures may peel off from the SERS substrate. A SERS sensing device and a method to fabricate such a device with an increased adhesion between the substrate and the plasmonic nanostructures to prevent loss of plasmonic nanostructures are presented in this disclosure.

In a **first** aspect of the disclosure, a method for manufacturing a SERS sensing device is presented. The method comprises: providing a silicon substrate 101, creating a silane layer 102 on a surface of the substrate 101, creating a plurality of plasmonic nanostructures 103 on the silane layer 102, characterized in that: creating a plurality of plasmonic nanostructures 103 on the silane layer 102 comprises completely covering the silane layer 102 with a metallic plasmonic layer 104.

The silane layer 102 may be created by immersing the silicon substrate 101 in a silane solution. The silane layer 102 functions as an adhesion layer between the silicon substrate 101 and the metallic plasmonic layer 104. In particular, the silane layer 102 provides a chemical bond between the plasmonic metallic layer 104 and the silicon substrate 101 wherein the silane layer 102 covalently binds to the silicon substrate 101 thereby forming Si-O-Si bonds. This covalent bond ensures integrity of the silane layer 102 during and after implantation in a human or animal body. As an advantage, the silane layer 102 does not provide any health hazards to the user as no peel off of the silane layer 102 will occur. The silane layer 102 also covalently binds to the metallic plasmonic layer 104. This covalent bond ensures integrity of the metallic plasmonic layer 104 during and after implantation in a human or animal body. As an advantage, the metallic plasmonic layer 104 does not provide any health hazards to the user as no peel off of the metallic plasmonic layer 104 will occur.

The silane layer 102 may be a mercapto-silane layer. It was observed that by using mercapto-silane as the silane layer, increased adhesion strength between the metallic plasmonic layer and the silicon can be obtained. The high adhesion strength ensures that no material will peel off from the silicon substrate during implantation or when implanted. Hence, the SERS sensing device can be considered as a bio-compatible device. Further, it was also observed that the use of mercapto silane does not influence SERS properties. This makes the mercapto-silane material ideal for use in a SERS sensor.

During experiments, the silane layer 102 was created on a surface of the silicon substrate 101 by immersing the silicon substrate 101 in a 10% (v/v) MPTES (3-mercaptopropyl-triethoxysilane) solution and in an ethanol and distilled water mixture (95:5 in volume). Thereafter, the silicon substrate was rinsed with ethanol and N2 to dry the silicon substrate. Thereafter, a baking step at 105 °C was performed on the silicon substrate in an N2 flowing oven for 10 minutes.

The metallic plasmonic layer 104 may be created by depositing a metallic plasmonic layer 104 on the silane layer 102 wherein the metallic plasmonic layer 104 completely covers the silane layer 102. The material of the metallic plasmonic layer 104 may be a metal with plasmonic properties such as gold, silver or copper. The metallic plasmonic layer 104 may be deposited on the silane layer 102 by evaporating a plasmonic metal on the silane layer. During experiments, a gold layer with a thickness of 30 nm was deposited on the silane layer 102 using a sputtering tool.

The deposition of the metallic plasmonic layer 104 is done after the silane layer is created but before any other steps of the method is performed on the silicon substrate. This allows the creation of a covalent bond between the complete metallic plasmonic layer 104 and the complete silane layer. As an advantage, the bond between the complete metallic plasmonic layer and the silane layer is very strong. This in contrast to prior art techniques whereby the metallic plasmonic layer is deposited only after patterning steps have been performed on the substrate. As a result of such a technique, the bond between the plasmonic structures and the silane layer is less strong, making these types of SERS substrates not suitable for implantation.

According to an embodiment of the disclosure, creating a plurality of plasmonic nanostructures 103 comprises: depositing a resist coating 105 on the metallic plasmonic layer 104, thereafter lithographically patterning the resist coating 105, thereafter etching the metallic plasmonic layer 104 and the silane layer 102 using the patterned resist coating as a mask,; thereafter cleaning the surface of the silicon substrate 101 to remove the patterned resist coating.

It is advantageous that the silane layer in between plasmonic nanostructures is removed as this reduces the possible binding of receptor molecules 109, e.g. molecules with a mercapto group, to that part of the silane layer. Hence, receptor molecules 109 only bind to the plasmonic nanostructures. This makes the SERS sensing device more sensitive.

The deposition of the resist coating 105 on the metallic plasmonic layer 104 may be done by coating the metallic plasmonic layer 104 with a negative photoresist material. During experiments, a gold layer was spin-coated with a 50 nm thick negative tone photoresist (ma-N-2400 from micro resist technology GmbH). Thereafter, the silicon substrate 101 was placed in an oven at 95 °C for 30 min.

The lithographical patterning of the resist coating 105 may be done by exposing parts of the resist coating 105 to an electron beam using a mask. Further, unexposed parts of the resist coating 105 may be removed using a developer solution thereby only leaving exposed resist coating 105 on the silicon substrate. During experiments, electron beam exposure was performed using electron beam lithography system (VB-HR from Leica) to create patterns. Thereafter, the patterns are developed using a developer solution (maD525 from micro resist technology GmbH). Thereafter, the silicon substrate was rinsed with water and N2 to dry.

The etching of the metallic plasmonic layer 104 and the silane layer 102 may be done using a dry etching technique wherein all material of the metallic plasmonic layer 104 and all material of the silane layer 102 are removed except the material underneath the created resist coating patterns. During experiments, gold and silane are etched using an ion miller.

The remaining material of the photoresist coating may be removed during a final cleaning step using a remover solution. During experiments, the remaining photoresist is removed using a remover solution (mr-rem 400 from micro resist technology GmbH).

Thereafter, the silicon substrate may be rinsed with water and N2 to dry.

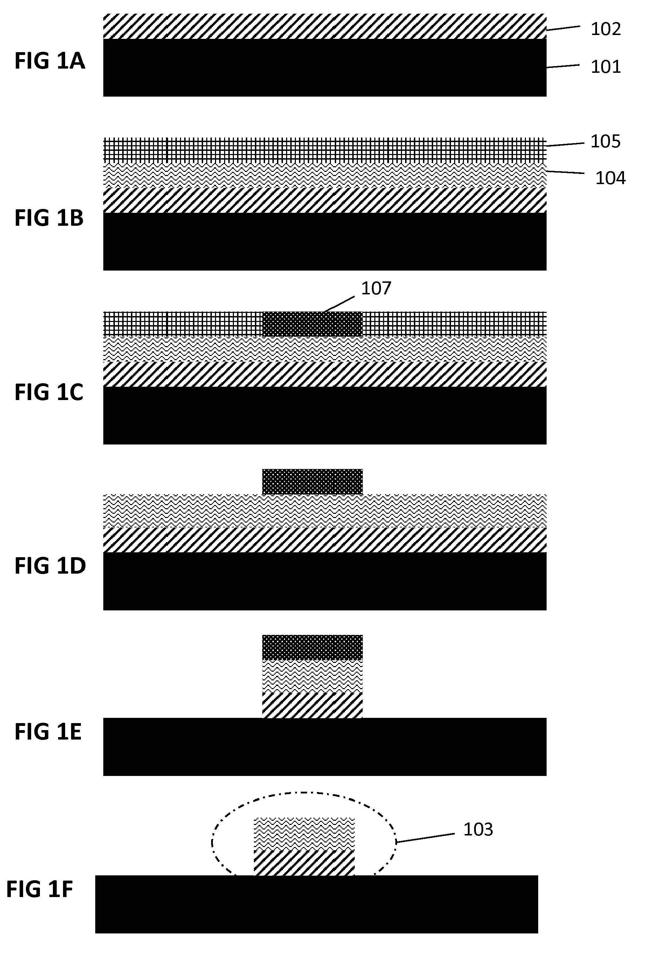

FIG 1A-FIG IF illustrate intermediate products of different steps of the method to create a SERS substrate.

FIG 1A illustrates a silicon substrate 101 and a silane layer 102 on top of a top surface of the silicon substrate 101.

FIG 1B illustrates the silane layer 102 completely covered with a metallic plasmonic layer 104. A photoresist coating 105 is located on top of the metallic plasmonic layer 104.

FIG 1C illustrates the silicon substrate after EV exposure. A part 107 of the photoresist coating was exposed to an electron beam.

FIG 1D illustrates the silicon substrate after removal of the unexposed photoresist coating.

FIG 1E illustrates the silicon substrate after removal of the metallic plasmonic layer 104 and the silane layer 102 except the material of the metallic plasmonic layer 104 and the silane layer 102 underneath the exposed photoresist coating 107.

**FIG IF** illustrates a SERS substrate whereby the exposed photoresist coating 107 is removed. An island containing a metallic plasmonic layer 104 and a silane layer 102 is located on top of the substrate 101. A SERS substrate may comprise a plurality of such islands wherein the size and position of each island is determined to create SERS hot-spots when irradiated.

According to an embodiment of the disclosure, steps of the method to fabricate the SERS substrate can be performed using CMOS compatible processing steps. As an advantage, the SERS substrate may be manufactured in mass production using existing CMOS manufacturing tools. This reduces the total cost of a SERS substrate.

According to an embodiment of the disclosure, creating the silane layer 102 comprises immersing the silicon substrate 101 in a silane solution for 2.5 to 17 hours.

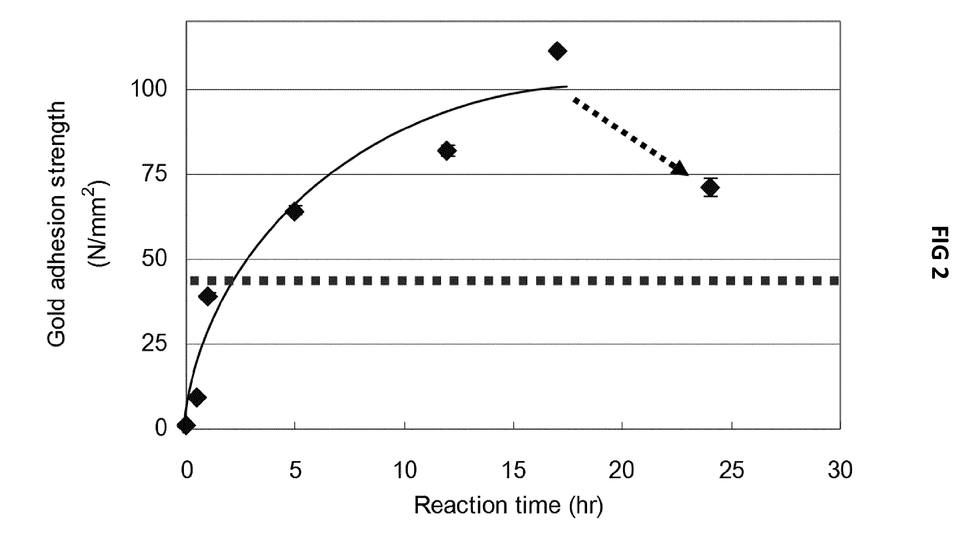

It was determined that for implantation purposes, the plasmonic nanostructure adhesion strength has to be at least 44 N/mm$^2$. It was discovered that the plasmonic nanostructure adhesion strength is directly related to the manufacturing process of the silane layer. During experiments, it was discovered that the reaction time for creating the silane layer is responsible for an increase of the adhesion strength of gold nanostructures while maintaining SERS properties. The reaction time is defined as the duration of immersing the silicon substrate in the silane solution. To achieve a gold adhesion strength of 44N/mm$^2$, experiments show that a reaction time of at least 2.5 hours to create the silane layer (silanization process) on the silicon substrate is required. Further, it was also discovered that when the reaction time is longer than 17 hours, the SERS properties and the adhesion strength of the gold nanostructures deteriorate due to the increased surface roughness created by the polymerisation of the silane layer on the silicon substrate. The relation between reaction time and gold adhesion strength is illustrated in FIG 2. The dotted line indicates the minimum required value of the adhesion strength of the plasmonic nanostructures for implantation purposes. The threshold is set at 44N/mm$^2$.

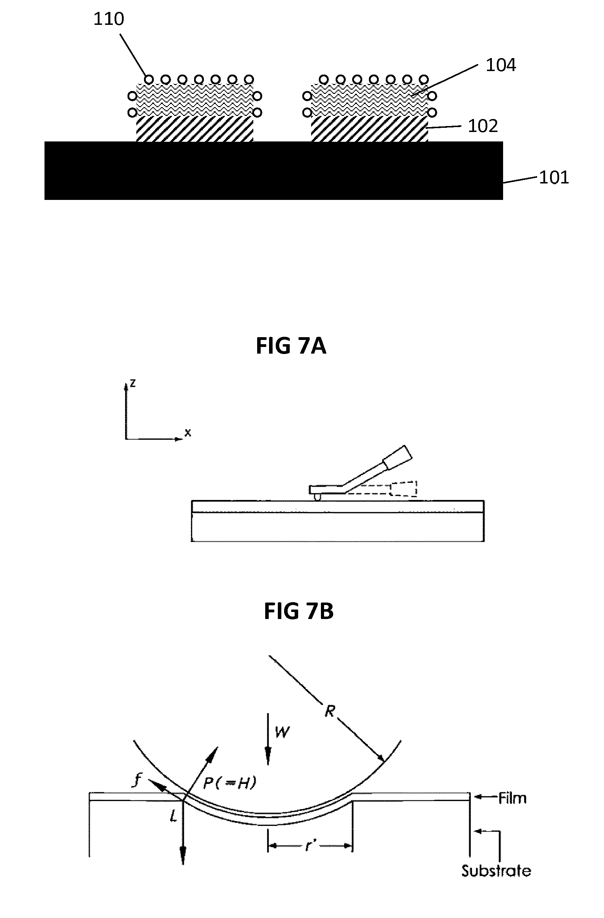

To determine the adhesion strength of plasmonic nanostructures on the silicon substrate, a scratch testing tool "CSR-2000 NANO · LAYER SCRATCH TESTER" produced by company RHESCA CO., LTD was used. The following measurement principle was used: A film surface (e.g. a surface comprising plasmonic nanostructures) is scratched in the X direction by a vibrating diamond stylus of a given curvature radius mounted on an elastic arm while the stylus is being lowered in the Z direction (load-increasing direction). The elastic arm is deformed because of the lowering movement of the stylus, resulting in the increased load to the film applied by the stylus (**FIG 7A**). Here, the load applied by the stylus to the film (W) and the shear stress on the boundary surface of the film (f), have the following relationship (**FIG 7B**): $F = \dfrac{H}{\sqrt{(\frac{\pi R^2 H}{W}) - 1}}$ where R is the curvature radius of the stylus, and H is the Brinell hardness of substrate. As the load to the film (W) is increased, the shear stress on the boundary surface (f) increases. The film breaks away when the shear stress exceeds the adhesive strength of the film. The product sheet of the scratch tool is publicly available.

The product obtained by the method described in the first aspect of the disclosure may be used as an in-vivo implantable device.

According to an embodiment of the disclosure, receptor molecules 109 for binding an analyte may be applied on the silicon substrate surface. This may be done by providing a solution comprising the receptor molecules 109 on the surface of the silicon substrate comprising the plasmonic nanostructures. These receptor molecules 109 may be molecules comprising a mercapto group enabling them to bind to a plasmonic metal. For example, a gold-sulphide (Au-S) bond is formed if the plasmonic metal is gold. However, this type of receptor molecule 109 also binds to the silane layer because disulphide bonds (S-S) are formed due to the oxidation of the mercapto group. It is therefore an advantage of the disclosure that the area of the silane layer is reduced as much as possible to allow the analyte to bind as close as possible to the plasmonic metal. As discussed earlier, the silane layer is not shared among different plasmonic nanostructures, receptor molecules 109 can thereby only bind to the plasmonic nanostructures. As an advantage, the sensitivity of the SERS sensing device is increased.

According to a specific embodiment of the disclosure, the receptor molecules 109 may be boronic acid derivate molecules for binding glucose molecules.

In a **second** aspect of the disclosure, a SERS sensing device is presented. The device comprises: a silicon substrate 101, a plurality of plasmonic nanostructures 103, 106 located on a surface 108 of the silicon substrate 101, each plasmonic nanostructure 103, 106 comprising a silane layer 102 and a metallic plasmonic layer 104, characterized in that: the silane layer 102 and the metallic plasmonic layer 104 of different plasmonic nanostructures 103, 106 are separated from each other, the silane layer (102) thereby being only present in between the silicon substrate (101) and the metallic plasmonic layer (104).

Each plasmonic nanostructure located on the silicon substrate surface may contain a silane layer 102 and a metallic plasmonic layer 104. The silane layer 102 of different plasmonic nanostructures 103, 106 is not shared among different plasmonic nanostructures. Each plasmonic nanostructure 103, 106 has its own silane layer 102 and its own metallic plasmonic layer 104. As discussed earlier, this is advantageous as an analyte cannot bind to receptor molecules 109 as no silane layer is present in between plasmonic nanostructures.

The size of the plasmonic nanostructures may be adapted to the analyte to be sensed. This is not possible when using a continuous metallic plasmonic layer as proposed in prior art documents. As an advantage, the SERS sensing device may feature a high sensitivity for a specific analyte. For example, the size of the plasmonic nanostructures may be adapted towards the sensing of a specific molecule, e.g. a glucose molecule. As a result, the SERS sensing device features a high sensitivity for the measuring of glucose. This is illustrated in FIG 6. The molecules 110 bind completely around the plasmonic nanostructure thereby increasing sensitivity of the SERS sensing device.

It is also noticed that using a rough continuous metallic plasmonic layer shows poor repeatability and uncontrollable behaviour due to the complexity of such a rough continuous metallic plasmonic layer. This in contrast to using plasmonic structures which show good repeatability and controllable behaviour due to the clearly defined size of each plasmonic nanostructure. This is advantageous as it allows the SERS sensing device to be used for the measuring of low concentrations of an analyte.

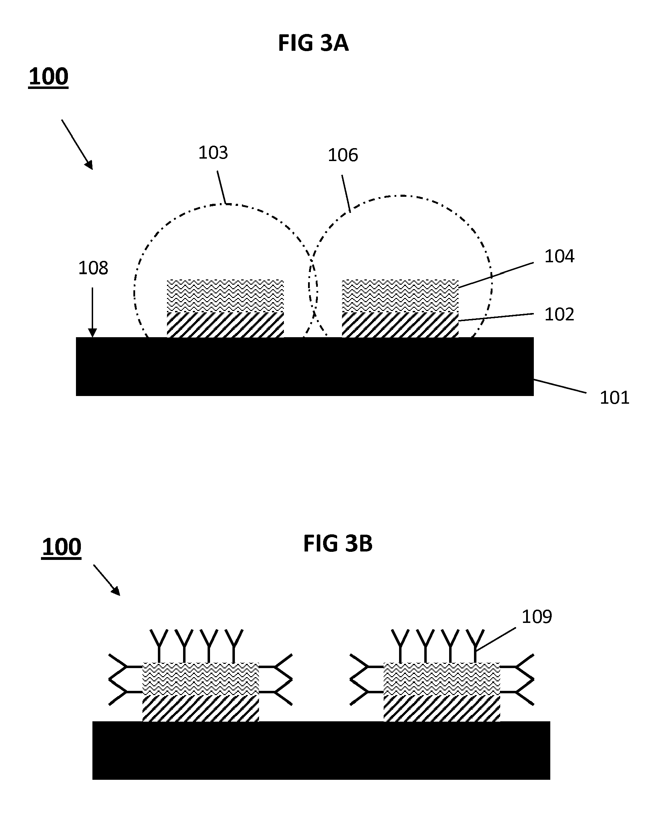

According to an embodiment of the disclosure, the plasmonic nanostructures may comprise receptor molecules 109 for binding an analyte. This is illustrated in **FIG 3B.** The receptor molecules 109 may be molecules which only bind to plasmonic nanostructures and not to the silicon based substrate. As an advantage, the sensitivity of the SERS sensing device is increased. The receptor molecule 109 may be a molecule comprising a mercapto-group enabling the molecule to bind to a plasmonic metal. For example, if the plasmonic metal is gold, an Au-S bond is formed between the gold and the molecule comprising a mercapto-group. The receptor molecule 109 may be selected based on the analyte to be sensed by the SERS sensing device. For example, if the analyte to be sensed is glucose, receptor molecule 109 maybe a boronic acid derivate.

The plurality of plasmonic nanostructures 103, 106 are configured to give rise to SERS when irradiated. The dimensions of each plasmonic nanostructure and the distance between plasmonic nanostructures may be determined to create SERS hot-spots when irradiated. For example, the diameter of each plasmonic nanostructure is 100 nm, the height of each plasmonic nanostructure is 30 nm and the distance between different plasmonic nanostructures is 600 nm.

**FIG 3A** illustrates an embodiment of the second aspect of the disclosure. Two islands 103, 106 are present on a top surface 108 of the silicon substrate 101; each island contains a silane layer 102 and a metallic plasmonic layer 104.

According to an embodiment of the disclosure, the adhesion strength between the metallic plasmonic layer 104 of each plasmonic nanostructure 103 and the silicon substrate 101 is at least $44N/mm^2$.

The SERS sensing device according to embodiments of the disclosure may be used as an in-vivo implantable device. The SERS sensing device according to embodiments of the disclosure may be used as an implantable sensor for measuring the concentration of an analyte in the bloodstream of an individual. For example, the SERS sensing device may be used to measure the concentration of glucose in the blood stream.

According to an embodiment of the disclosure, the silicon substrate surface comprises a plurality of groups of plasmonic nanostructures. Each group comprises a plurality of plasmonic nanostructures 103. For example, the area of a group of plasmonic nanostructures is $1 mm^2$, the diameter of one plasmonic nanostructure is 100 nm, the distance in between plasmonic nanostructures is 600 nm and the height of each plasmonic nanostructure is 30 nm.

It was observed that for SERS devices manufactured according to this disclosure, after an implantation period of 2 months in an animal body the different groups of plasmonic structures were still present and individual plasmonic nanostructures remained intact.

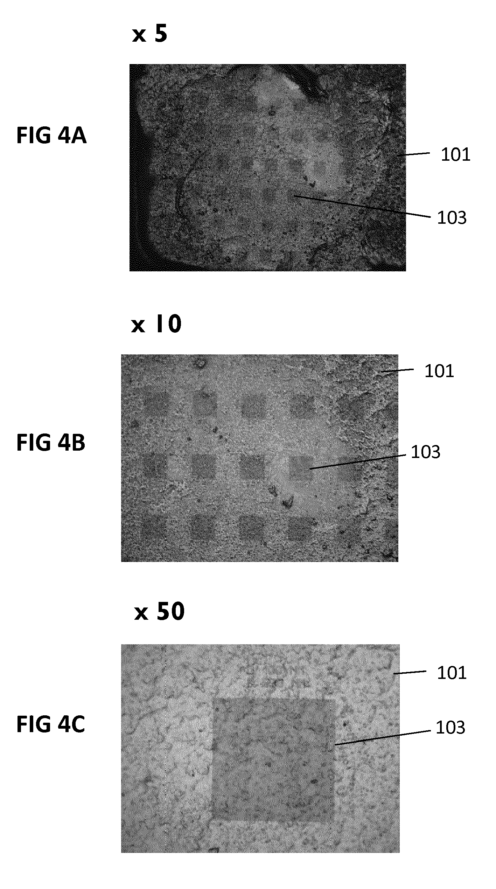

A bright field image of different groups of plasmonic nanostructures is illustrated in **FIG 4B**. One group of plasmonic nanostructures is illustrated in SEM image **FIG4C.**

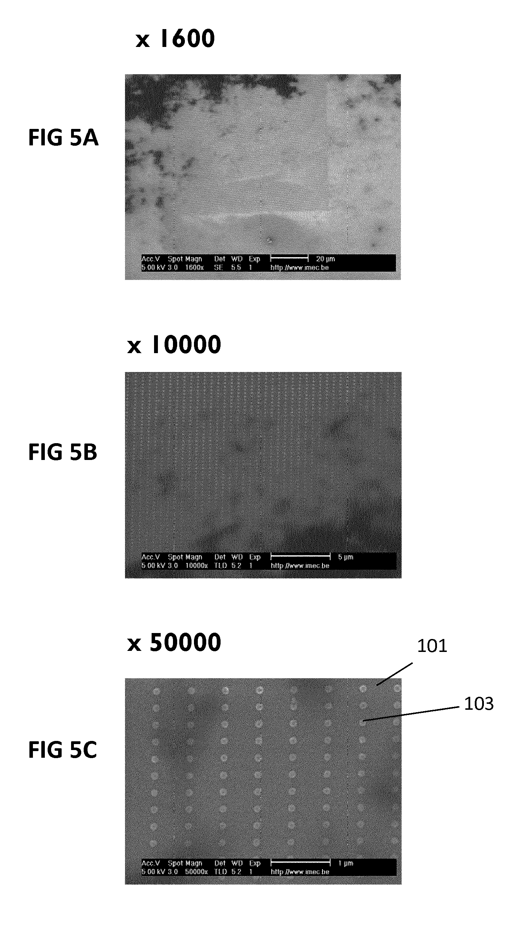

**FIG 4a, 4B, 4C, 5A, 5B, 5C** are images of the same SERS sensing device recorded after an implantation period of 2 months in an animal. **FIG 4A, 4B and 4C** are bright field images of the SERS sensing device, with a magnification factor of respectively 5, 10 and 100. **FIG 5A, 5B and 5C** are SEM images of the SERS sensing device with a magnification factor of respectively 1600, 10000 and 50000.

**Claims**

1. A method for manufacturing a SERS sensing device (100), the method comprising:

   - providing a silicon substrate (101),
   - creating a silane layer (102) on a surface of the silicon substrate (101), and
   - creating a plurality of plasmonic nanostructures (103) on the silane layer (102),
   **characterized in that**:

   creating a plurality of plasmonic nanostructures (103) on the silane layer (102) comprises completely covering the silane layer (102) with a metallic plasmonic layer (104).

2. The method according to claim 1 wherein creating the silane layer (102) comprises immersing the substrate (101) in a silane solution for 2,5 to 17 hours.

3. The method according to any of the preceding claims wherein creating a plurality of plasmonic nanostructures (103) further comprises:

- depositing a resist coating (105) on the metallic plasmonic layer (104), thereafter
- lithographically patterning the resist coating (105), thereafter
- etching the metallic plasmonic layer (104) and the silane layer (102) using the patterned resist coating as a mask, and thereafter
- removing the patterned resist coating.

4. The method according to any of the preceding claims, further comprising depositing receptor molecules (109) on the surface of the silicon substrate (101), the receptor molecules (109) being configured to bind to plasmonic nanostructures (103) and configured to bind an analyte.

5. The method according to claim 4 wherein the analyte is glucose and wherein the receptor molecules (109) are boronic acid derivatives.

6. The method according to any of the preceding claims, wherein the silane layer (102) is a mercapto-silane layer.

7. The method according to any of the preceding claims wherein the metallic plasmonic layer (104) is a gold, silver or copper layer.

8. An implantable SERS sensing device (100), the device comprising:

- a silicon substrate (101);
- a plurality of plasmonic nanostructures (103, 106) located on a surface (108) of the silicon substrate (101), each plasmonic nanostructure (103, 106) comprising a silane layer (102) and a metallic plasmonic layer (104);
**characterized in that**:

the silane layer (102) and the metallic plasmonic layer (104) of different plasmonic nanostructures (103, 106) are separated from each other, the silane layer (102) thereby being only present in between the silicon substrate (101) and the metallic plasmonic layer (104).

9. The implantable SERS sensing device (100) according to claim 8 wherein the adhesion strength between the metallic plasmonic layer (104) of each plasmonic nanostructure (103) and the silicon substrate (101) is at least 44N/mm2.

10. The implantable SERS sensing device (100) according to any of claims 8 to 9, wherein the silane layer is a mercapto-silane layer.

11. The implantable SERS sensing device (100) according to any of claims 8 to 10, wherein a plasmonic nanostructure (103) comprises a receptor molecule (109) configured to bind an analyte.

12. The implantable SERS sensing device (100) according to claim 11, wherein the analyte is glucose and wherein the receptor molecule (109) is a boronic acid derivative.

13. The implantable SERS sensing device according to any of claims 8 to 12, wherein the metallic plasmonic layer (104) is a gold, silver or copper layer.

14. Use of the SERS sensing device (100) according to any of claims 8 to 13 for measuring the concentration of an analyte in blood.

15. Use of the SERS sensing device (100) according to any of claims 8 to 13 for measuring the concentration of glucose in blood.

FIG 1A

FIG 1B

FIG 1C

FIG 1D

FIG 1E

FIG 1F

# FIG 2

## FIG 3A

100

## FIG 3B

100

**x 5**

**FIG 4A**

— 101

— 103

**x 10**

**FIG 4B**

— 101

— 103

**x 50**

**FIG 4C**

— 101

— 103

x 1600

FIG 5A

x 10000

FIG 5B

x 50000

FIG 5C

**FIG 7A**

**FIG 7B**

Europäisches
Patentamt

European
Patent Office

Office européen
des brevets

# EUROPEAN SEARCH REPORT

Application Number

EP 15 15 1213

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2009/066946 A1 (WANG HONG [US] ET AL) 12 March 2009 (2009-03-12) <br> * figures 18,19,24 * <br> * paragraph [0018] * <br> * paragraph [0089] - paragraph [0095] * <br> ----- | 1-15 | INV. <br> G01N21/552 <br> G01N21/65 |
| Y | US 6 406 777 B1 (BOSS PAMELA A [US] ET AL) 18 June 2002 (2002-06-18) <br> * figures 3,4 * <br> * column 3, line 19 - column 4, line 67 * <br> ----- | 1-15 | |
| Y | EP 2 442 142 A1 (UNIV TROYES TECHNOLOGIE [FR]) 18 April 2012 (2012-04-18) <br> * claims 1-11 * <br> ----- | 1-15 | |
| Y | MAROUA BEN HADDADA ET AL: "Optimizing the immobilization of gold nanoparticles on functionalized silicon surfaces: amine- vs thiol-terminated silane", GOLD BULLETIN, vol. 46, no. 4, 17 December 2013 (2013-12-17), pages 335-341, XP055188407, ISSN: 0017-1557, DOI: 10.1007/s13404-013-0120-y <br> * abstract * <br> * page 336, last paragraph - page 337, paragraph first paragraph * <br> ----- | 1-15 | |
| | | | TECHNICAL FIELDS SEARCHED (IPC) <br><br> G01N |
| A | ALEX STEPHENSON-BROWN ET AL: "Glucose selective Surface Plasmon Resonance-based bis-boronic acid sensor", THE ANALYST, vol. 138, no. 23, 1 January 2013 (2013-01-01), page 7140, XP055188538, ISSN: 0003-2654, DOI: 10.1039/c3an01233f <br> * abstract * <br> * paragraph [introduction] * <br> ----- | 4,5,11, 12,14 | |

The present search report has been drawn up for all claims

| Place of search | Date of completion of the search | Examiner |
|---|---|---|
| The Hague | 21 May 2015 | Rasmusson, Marcus |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.** EP 15 15 1213

This annex lists the patent family members relating to the patent documents cited in the above-mentioned European search report.
The members are as contained in the European Patent Office EDP file on
The European Patent Office is in no way liable for these particulars which are merely given for the purpose of information.

21-05-2015

| Patent document cited in search report | | Publication date | Patent family member(s) | | Publication date |
|---|---|---|---|---|---|
| US 2009066946 | A1 | 12-03-2009 | CN | 101400976 A | 01-04-2009 |
| | | | US | 2007153267 A1 | 05-07-2007 |
| | | | US | 2009066946 A1 | 12-03-2009 |
| | | | US | 2010110424 A1 | 06-05-2010 |
| | | | WO | 2007149120 A2 | 27-12-2007 |
| US 6406777 | B1 | 18-06-2002 | US | 6406777 B1 | 18-06-2002 |
| | | | US | 6776962 B1 | 17-08-2004 |
| EP 2442142 | A1 | 18-04-2012 | EP | 2442142 A1 | 18-04-2012 |
| | | | FR | 2965749 A1 | 13-04-2012 |
| | | | US | 2012121870 A1 | 17-05-2012 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

• New Gold Nanoparticles Adhesion Process Opening the Way of Improved and Highly Sensitive Plasmonics Technologies. *Plasmonics,* 2013, vol. 8, 411-415